# EUROPEAN PATENT SPECIFICATION

(11) **EP 4 463 207 B1**
(45) Date of publication and mention of the grant of the patent: **15.04.2026**
(21) Application number: 23705753.4
(22) Date of filing: 17.01.2023
(51) Int. Cl.: A61M 25/00, A61M 25/06

(54) **METHOD FOR FORMING RAPIDLY INSERTABLE CENTRAL CATHETERS (RICC) AND DEVICE**
VERFAHREN ZUR BILDUNG VON SCHNELL EINSETZBAREN ZENTRALKATHETERN (RICC) UND VORRICHTUNG
PROCÉDÉ DE FORMATION DE CATHÉTERS CENTRAUX À INSERTION RAPIDE (RICC) ET DISPOSITIF.

(30) Priority: 21.01.2022 US 202263301886 P
(43) Date of publication of application: 20.11.2024
(73) Proprietor: Bard Access Systems, Inc., Salt Lake City, UT 84116 (US)
(72) Inventor: HOWELL, Glade H., Draper, UT 84020 (US)
(74) Representative: Hoffmann Eitle
(86) International application number: PCT/US2023/010972
(87) International publication number: WO 2023/141112

(56) References cited:
- WO-A1-2016/123278
- US-A1- 2021 121 661
- US-A1- 2022 001 138

## Description

### PRIORITY

This application claims the benefit of priority to U. S. Provisional Application No. 63/301,886, filed January 21, 2022.

### BACKGROUND

Rapidly Insertable Central Catheter ("RICC") systems include a RICC catheter having a catheter body defining two or more lumen, and an access section extending therefrom and defining a single lumen. The RICC further includes a dilator section disposed between the access section and the catheter body section and defines a tapered outer profile. The configuration of the RICC allows a clinician to perform the steps of accessing the vasculature, dilating the access site, and placing the catheter body defining one or more lumens, in a single action. Under conventional catheter placement techniques, such steps would have to be performed separately and require the repeated introduction and removal of several tools and medical devices. As such RICC systems simplify complicated and laborious placement techniques, mitigate the introduction of pathogens, and reduce the overall procedure duration.

However, forming the RICC catheter, which is required to perform several different functions, requires coupling the three different structures of the catheter body, dilator section, and access section together, while maintaining a smooth abluminal surface. Each of the three different structures are required to display different mechanical properties to fulfil the respective roles in the placement process, and as such, are often formed of different materials. Conventional manufacturing techniques results in a bump or ridge being formed adjacent a join between two or more of these structures. Where the bump occurs on an outer surface, the bump can catch a portion of the skin at the insertion site inhibiting the distal advancement of the RICC and disrupting the placement procedure. Where the bump occurs within a lumen, the bump can snag the tip of a guidewire or the like, inhibiting movement of the guidewire therethrough. Additional machining or milling procedures are required to remove such bumps or ridges and provide a smooth surface, but such secondary manufacturing procedures increase costs and complexity.

Further, conventional manufacturing techniques often require secondary machining or milling procedures to form the apertures communicating with the lumen. Either the apertures are formed in a side wall or in a distal end of the catheter, after the catheter has been formed. Forming apertures in a tapered outer surface while maintaining a smooth abluminal surface can be particularly challenging. Errors in these secondary machining procedures can lead to wastage in the manufacturing process, or failure of the device. As such apertures are often formed distally or proximally of the tapered sections and formed perpendicular to the axis of the lumen.

US 2022/001138 A1 discloses rapidly insertable central catheters and methods thereof.

### SUMMARY

Briefly summarized, embodiments disclosed herein are directed to distal tip structures for a catheter, such as a rapidly insertable central catheter (RICC), and associated methods of manufacture. Embodiments are directed to forming intricate distal tip structures, including one or more different materials, while improving manufacturing efficiencies. Embodiments are directed to providing a RICC distal tip structure having a smooth abluminal and intra-luminal surfaces, with little or no secondary machining procedures to remove ridges or bumps, or forming apertures. Further, embodiments are directed to forming apertures in tapered surfaces during the primary manufacturing step. Such distal tip structures reduce a longitudinal length between the apertures and a distal tip and improve the efficacy of device. Additionally, reducing the number of manufacturing steps reduces costs and wastage, and improves manufacturing efficiencies. The invention is defined in claims 1,13. Preferable embodiments are defined in the dependent claims.

Disclosed herein is a method of forming a rapidly insertable central catheter including, providing a catheter body section having a primary lumen, a secondary lumen and a tertiary lumen, each extending longitudinally, placing a distal end of the catheter body within a die, the die having a first mandrel engaging the primary lumen, a second mandrel engaging the secondary lumen, and a third mandrel engaging the tertiary lumen, melting a polymeric material disposed within a cavity of the die to form a dilator section coupled to the distal tip of the catheter body, the dilator section defining a tapered out profile and including a secondary lumen aperture disposed in a side wall and communicating with the secondary lumen, and a tertiary lumen aperture disposed in a side wall and communicating with the tertiary lumen.

In some embodiments, the method further includes forming an access section integrally with the dilator section and extending from a distal end thereof, the access section defining a single lumen communicating with primary lumen and extending to a primary lumen aperture disposed at a distal tip thereof.

In some embodiments, wherein the access section and the dilator section are formed of a first polymeric material and the catheter body is formed of a second polymeric material, the first polymeric material displaying a harder durometer relative to the second polymeric material.

In some embodiments, wherein the first mandrel includes a collar disposed at a distal end thereof, the collar defining an outer diameter equal to an outer diameter of an access section.

In some embodiments, the method further includes placing a proximal end of the access section within a receptacle of the dilator section, defined by the collar of the first mandrel and securing the access section to the dilator section using adhesive, bonding, solvent bonding, or welding.

In some embodiments, the access section is formed of a first polymeric material and the catheter body is formed of a second polymeric material, the first polymeric material displaying a harder durometer relative to the second polymeric material.

In some embodiments, the dilator section is formed of one of the first polymeric material, the second polymeric material, or a third polymeric material having a harder durometer relative to the second polymeric material and a softer durometer relative to the first polymeric material.

In some embodiments, the melting step further includes applying one of radio frequency (RF) energy or thermal energy to melt the polymeric material.

In some embodiments, one or both of the secondary lumen aperture and the tertiary lumen aperture are disposed in a longitudinal mid-point of the dilator section between a proximal end and a distal end.

In some embodiments, the secondary lumen aperture and the tertiary lumen aperture are disposed at an equal longitudinal length relative to a distal tip of the rapidly insertable central catheter.

In some embodiments, the method further includes forming an introducing aperture disposed proximally of one or both of the secondary lumen aperture and the tertiary lumen aperture and communicating with the primary lumen, the introducing aperture configured to receive an access needle therethrough, a distal tip of the access needle extending through a primal lumen aperture disposed at a distal tip of the rapidly insertable central catheter.

In some embodiments, providing a catheter body further includes extruding or trimming the catheter body section to a predetermined length and coupling a proximal end of the catheter body to one or more of a catheter hub and one or more extension legs.

Also disclosed is a rapidly insertable central catheter system including, a rapidly insertable central catheter having, a catheter body defining a primary lumen, a secondary lumen, and a tertiary lumen, and including an introducing aperture disposed in a side wall and communicating with the primary lumen, a dilator section extending distally from the catheter body and defining a tapered outer profile, the dilator section defining a secondary lumen aperture disposed in a side wall and communicating with the secondary lumen and defining a tertiary lumen aperture disposed in a side wall and communicating with the tertiary lumen, and an access section extending from the dilator section and including a primary lumen aperture disposed at a distal tip thereof and communicating with the primary lumen, and a needle extending through the introducing aperture and through a distal portion of the primary lumen, a distal tip of the needle extending through the primary lumen aperture.

In some embodiments, the secondary lumen aperture and the tertiary lumen aperture are disposed at an equal longitudinal length from a distal tip of the access section.

In some embodiments, a longitudinal length of the access section is less than a longitudinal length of the dilator section.

In some embodiments, the access section is formed of a first material and the catheter body is formed of a second material having a softer durometer relative to the first material.

In some embodiments, the dilator section is formed of one of the first material, the second material or a third material having a harder durometer relative to the second material and a softer durometer relative to the first material.

In some embodiments, the dilator section and the access section are formed integrally as a single monolithic piece and fused with the catheter body using RF welding.

In some embodiments, the dilator section is fused with the catheter body using RF welding and defines a receptacle at a distal end, the receptacle having a diameter equal to an outer diameter of the access section, a proximal end of the access section coupled with the receptacle using adhesive, bonding, solvent bonding, or welding.

These and other features of the concepts provided herein will become more apparent to those of skill in the art in view of the accompanying drawings and following description, which describe particular embodiments of such concepts in greater detail.

### DRAWINGS

FIG. 1 shows a side view of an exemplary RICC placement system, in accordance with examples disclosed herein.
FIG. 2 shows close up detail of a distal portion of the RICC of FIG. 1, in accordance with examples disclosed herein.
FIGS. 3A-3D show various cross-sectional views of the distal portion of FIG. 2, in accordance with examples disclosed herein.
FIG. 4A shows a perspective view of a RICC placement system, in accordance with embodiments disclosed herein.
FIG. 4B shows a side view of the RICC placement system of FIG. 4A, in accordance with embodiments disclosed herein.

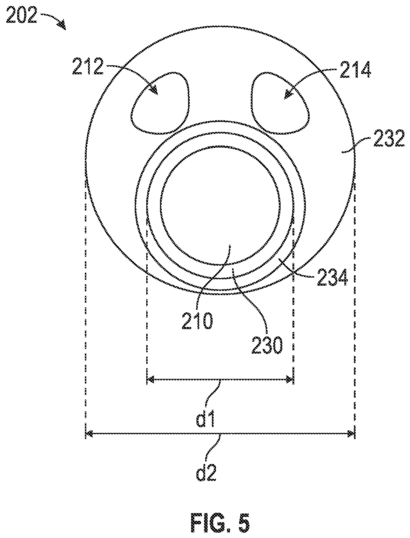

FIG. 5 shows a distal end view of the RICC of the RICC placement system of FIG. 4A, in accordance with embodiments disclosed herein.
FIG. 6A shows a perspective view of a distal portion of a RICC, in accordance with embodiments disclosed herein.
FIG. 6B shows a plan view of a distal portion of a RICC, in accordance with embodiments disclosed herein.
FIG. 6C shows longitudinal cross-section view of a RICC, in accordance with embodiments disclosed herein.
FIG. 7A shows a longitudinal vertical cross-section view of a die, in accordance with embodiments disclosed herein.
FIG. 7B shows a longitudinal horizontal cross-section view of the die of FIG. 7A, in accordance with embodiments disclosed herein.
FIG. 7C shows a lateral cross-section view of the die of FIG. 7A, in accordance with embodiments disclosed herein.
FIGS. 8A-8F show an exemplary method of forming a catheter tip, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

In the following description, the terms "or" and "and/or" as used herein are to be interpreted as inclusive or meaning any one or any combination. As an example, "A, B or C" or "A, B and/or C" mean "any of the following, A, B, C, A and B, A and C, B and C, A, B and C." An exception to this definition will occur only when a combination of elements, components, functions, steps or acts are in some way inherently mutually exclusive.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

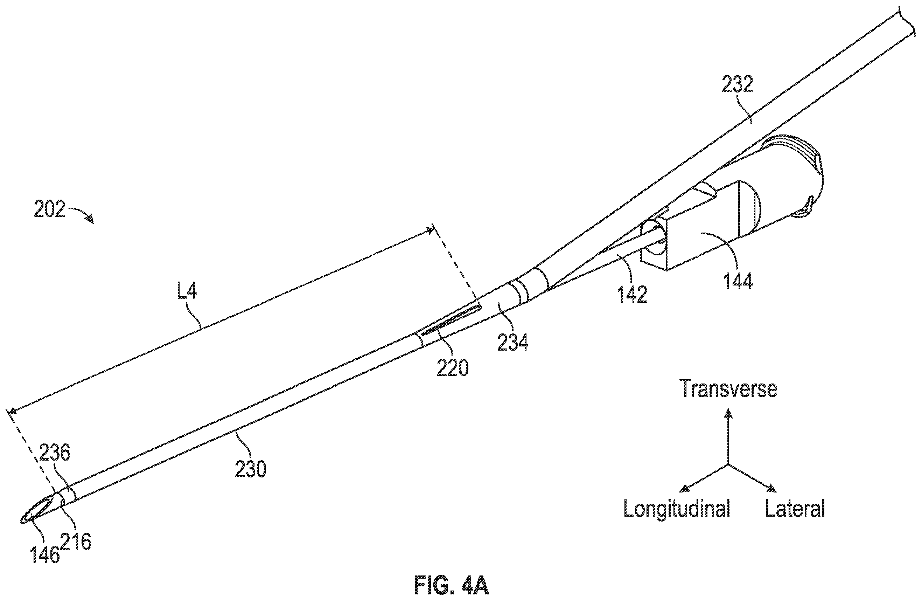

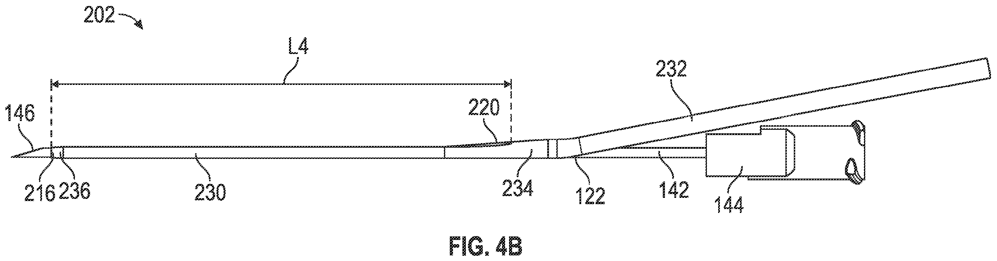

To assist in the description of embodiments described herein, as shown in FIG. 4A, a longitudinal axis extends substantially parallel to an axial length of the catheter. A lateral axis extends normal to the longitudinal axis, and a transverse axis extends normal to both the longitudinal and lateral axes. A horizontal plane is defined by the longitudinal and lateral axes. A vertical plane extends normal to the horizontal plane.

Unless defined otherwise, all technical and scientific terms used herein have the same meaning as commonly understood by those of ordinary skill in the art.

As discussed herein, there is a need to overcome certain artifacts in the manufacturing of rapidly insertable central catheters (RICC). For example, a RICC can include a multiluminal, catheter body section, and can transition to a monoluminal access section adjacent a distal end thereof. The transition between these sections can be considerably difficult to manufacture without creating an edge or bump on an outer surface of the RICC that catches the skin of a patient around an insertion site and obstructs placement of the RICC subcutaneously. Therefore, also disclosed herein are RICCs and methods of manufacture thereof that address the foregoing need.

### Rapidly insertable central catheter systems

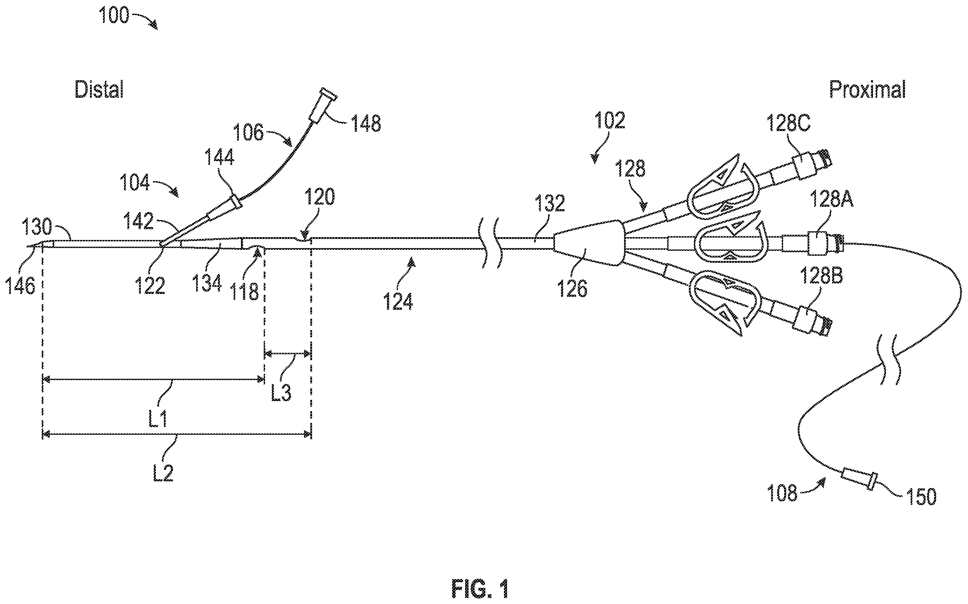

FIGS. 1-3D illustrate an exemplary rapidly insertable central catheter ("RICC") placement system ("system") 100 not forming part of the invention. As shown, the system 100 generally includes a rapidly insertable central catheter ("RICC") 102, an introducer needle ("needle") 104, and one or more guidewires, for example, an access guidewire 106, and a placement guidewire 108.

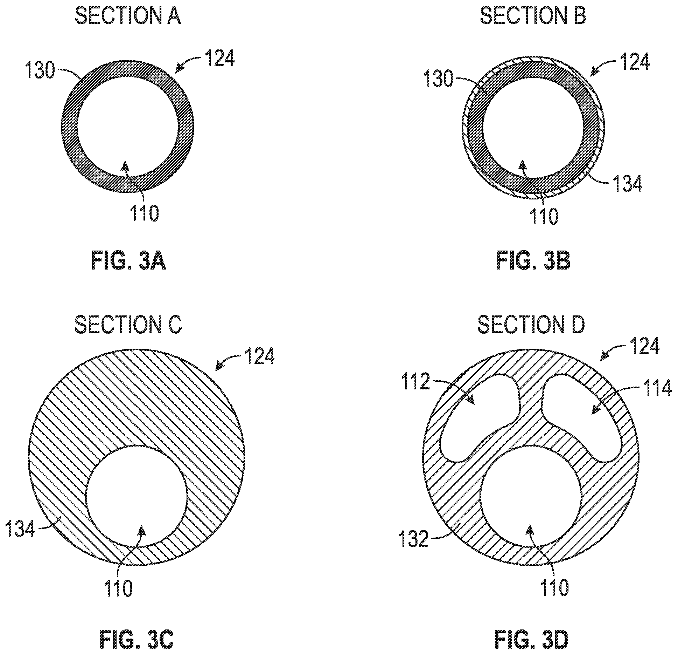

In an example, the RICC 102 can be a monoluminal or multiluminal RICC (e.g., a diluminal RICC, a triluminal RICC, a tetraluminal RICC, a pentaluminal RICC, a hexaluminal RICC, etc.). As shown in FIGS. 1 and 3D, the RICC 102 is a triluminal RICC including a set of three lumens. Such a set of three lumens includes a primary lumen 110 (e.g., a distal lumen), a secondary lumen 112 (e.g., a medial lumen), and a tertiary lumen 114 (e.g., a proximal lumen).

As shown in FIG. 1, the RICC 102 further includes a hub 126 disposed at a proximal end, and one or more extension legs 128 extending proximally from the hub 126. Each extension leg 128 is in fluid communication with a lumen 110, 112, or 114 of the RICC 102. For example, as shown, a first extension leg 128A is in fluid communication with the distal lumen 110, a second extension leg 128B is in fluid communication with the medial lumen 112, and a third extension leg 128C is in fluid communication with the proximal lumen 114.

Whether the RICC 102 is monoluminal or multiluminal, the RICC 102 includes at least the primary lumen 110. The primary lumen 110 typically extends from a proximal end of the RICC 102 to a distal end of the RICC 102 such as from an opening of a corresponding Luer connector to a primary-lumen aperture 116 in a distal end of the catheter tube 124 (e.g., the hard portion of the catheter tube 124 or the first section (access section 130) of the catheter tube 124) set forth herein. When the RICC 102 has two or more lumens, the RICC 102 further includes at least the secondary lumen 112. The secondary lumen 112 typically extends from the proximal end of the RICC 102 to a distal portion of the RICC 102 such as from an opening of a corresponding Luer connector to a secondary-lumen aperture 118 in the distal portion of the catheter tube 124 (e.g., the soft portion of the catheter tube 124 or the second section (catheter body 132) of the catheter tube 124) proximal of the primary-lumen aperture 116.

When the RICC 102 has three or more lumens, the RICC 102 further includes at least the tertiary lumen 114. The tertiary lumen 114 typically extends from the proximal end of the RICC 102 to the distal portion of the RICC 102 such as from an opening of a corresponding Luer connector to a tertiary-lumen aperture 120 in the distal portion of the catheter tube 124 (e.g., the soft portion of the catheter tube 124 or the first section 132 of the catheter tube 124) proximal of the secondary-lumen aperture 118. Notwithstanding the foregoing, each lumen of the secondary lumen 112 and the tertiary lumen 114 can distally extend slightly farther than the secondary-lumen aperture 118 and the tertiary-lumen aperture 120, respectively, in view of different manufacturing methods.

In an example, a longitudinal length between a primary-lumen aperture 116 and a secondary-lumen aperture 118 can be a first length (*L1*). In an example, a longitudinal length between a primary-lumen aperture 116 and a tertiary-lumen aperture 120 can be a second length (*L2*). In an example, the first length (*L1*) can be greater than the second length (*L2*). As such a difference between the first length (*L1*) and the second length (*L2*) can be a third length (*L3*)*.* In an example, the first length (*L1*) or the second length *(L2)* can be between 3cm and 10cm. In an example the first length (*L1*) or the second length (*L2*) can be substantially 7cm. However it will be appreciated that greater or lesser lengths are also contemplated.

In an example, the RICC 102 includes an introducing aperture 122, which is dedicated to accommodating insertion of the introducer needle 104 therethrough for coupling the RICC 102 and the introducer needle 104 together in the system 100. However, the RICC 102 need not include a dedicated introducing aperture such as the introducing aperture 122. Indeed, the secondary-lumen aperture 118 or the tertiary-lumen aperture 120 can serve as the introducing aperture 122 in some examples, which requires a different mode of coupling the RICC 102 and the introducer needle 104. Alternatively, such as when the RICC 102 is monoluminal, an introducing aperture can be created as needed by puncturing the catheter tube 124 with the introducer needle 104.

The RICC 102 further includes an introducing lumen coincident with a distal portion of the primary lumen 110. In other words, the introducing lumen is an introducing portion of the primary lumen 110 of the RICC 102. In the RICC 102, the introducing lumen is the distal portion of the primary lumen 110 extending from the introducing aperture 122 to the primary-lumen aperture 116. The introducing aperture 122, which can be distal of the secondary-lumen aperture 118, between the secondary-lumen aperture 118 and the tertiary-lumen aperture 120, or proximal of the tertiary-lumen aperture 120, opens directly into a proximal end of the introducing portion of the primary lumen 110 of the RICC 102. For a RICC without the introducing aperture 122, the introducing lumen is the distal portion of the primary lumen 110 extending from the secondary-lumen aperture 118, the tertiary-lumen aperture 120, or a puncture created with the introducer needle 104 to the primary-lumen aperture 116. Whether the introducing lumen extends from the secondary-lumen aperture 118, the tertiary-lumen aperture 120, or the puncture depends upon which aperture of the foregoing apertures accommodates the introducer needle 104. Neither the secondary-lumen aperture 118 nor the tertiary-lumen aperture 120 opens directly into a proximal end of the introducing portion of the primary lumen 110 of the RICC without the introducing aperture 122. Instead, the introducer needle 104 pierces a septum between the secondary lumen 112 or the tertiary lumen 114 and the primary lumen 110 respectively by way of the secondary-lumen aperture 118 or the tertiary-lumen aperture 120.

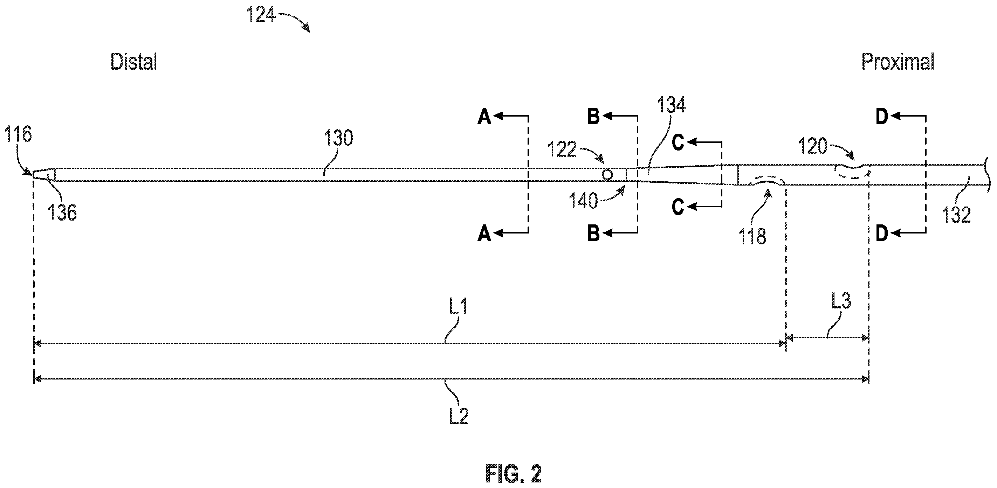

FIG. 2 illustrates a distal portion of the catheter tube 124 of the RICC 102 of FIG. 1 in accordance with some exaamples. FIGS. 3A-3D illustrate various transverse cross sections of the catheter tube 124 of FIG. 2 in accordance with some examples.

The catheter tube 124 includes a hard portion of the catheter tube 124 and a soft portion of the catheter tube 124, wherein "hard" and "soft" are used in a relative sense in that the hard portion of the catheter tube 124 is harder than the soft portion of the catheter tube 124. The hard potion of the catheter tube 124 includes a first, monoluminal section, or "access section" 130 in a distal portion of the catheter tube 124, and the soft potion of the catheter tube 124 includes a second section, or "catheter body" 132 having one or more lumens and extending from a proximal portion of the catheter tube 124 to the distal portion thereof but proximal of the access section 130. In an example, a dilation section 134, or dilator section 134 of the catheter tube 124 can be formed of the same material as the access section 130 and can display the same mechanical properties thereof. In an example, the dilator section 134 of the catheter tube 124 can be formed of the same material as the catheter body section 132 and can display the same mechanical properties thereof. In an example, the dilator section 134 of the catheter tube 124 can be formed of a third material different from that of the first material of the access section 130 and the second material of the catheter body section 132. In an example, the third material can be softer than the relatively hard first material and harder than the relatively soft second material. In an example, the third material can be softer than the second material of the catheter body section 132. In an embodiment, the third material can be harder than the first material of the access section 130.

In an example, the foregoing arrangement of the access section 130 of the catheter tube 124, the catheter body section 132 of the catheter tube 124, and the dilator section 134 possess a column strength sufficient to prevent buckling of the catheter tube 124 when inserted into an insertion site established by a percutaneous puncture and advanced through a vasculature of a patient. As set forth herein, the catheter tube 124 is shown with the introducing aperture 122 through a side of the catheter tube 124 in a distal portion thereof, e.g. an access section 130.

The access section 130 of the catheter tube 124 is in the distal portion of the catheter tube 124. The access section 130 includes a distal tip 136 having a relatively short taper continuing from the beveled tip 146 of the introducer needle 104 in the system 100 to an outer diameter of a remainder of the access section 130. The taper of the distal tip 136 is configured for immediate dilation of tissue about a percutaneous puncture established with the introducer needle 104 up to the outer diameter of the remainder of the access section 130 of the catheter tube 124. The access section 130 also includes a proximal portion disposed in the receptacle 138 of the dilator section 134 and fixedly coupled (e.g., solvent bonded, adhered, welded, etc.) thereto.

The access section 130 of the catheter tube 124 is formed of a first polymeric material having a first durometer. The first polymeric material can be polytetrafluoroethylene, polypropylene, or polyurethane, but the first polymeric material is not limited to the foregoing polymers. Polyurethane is advantageous in that the access section 130 of the catheter tube 124 can be relatively rigid at room-temperature but become more flexible *in vivo* at body temperature, which reduces irritation to vessel walls and phlebitis.

The catheter body section 132 of the catheter tube 124 extends from the proximal portion of the catheter tube 124 to the distal portion thereof but proximal of the access section 130 of the catheter tube 124. In an example, the catheter body section 132 includes a distal end integral (e.g., RF welded) with a proximal end of the dilator section 134 and a proximal portion disposed in the catheter hub 126 and fixedly coupled (e.g., solvent bonded, welded, adhered, etc.) thereto.

The catheter body section 132 of the catheter tube 124 is formed of a second polymeric material having a second durometer less than the first durometer. The second polymeric material can be polyvinyl chloride, polyethylene, polyurethane, or silicone, but the second polymeric material is not limited to the foregoing polymers. In addition to that set forth above for polyurethane in the access section 130 of the catheter tube 124, polyurethane is advantageous in that it can be less thrombogenic than some other polymers.

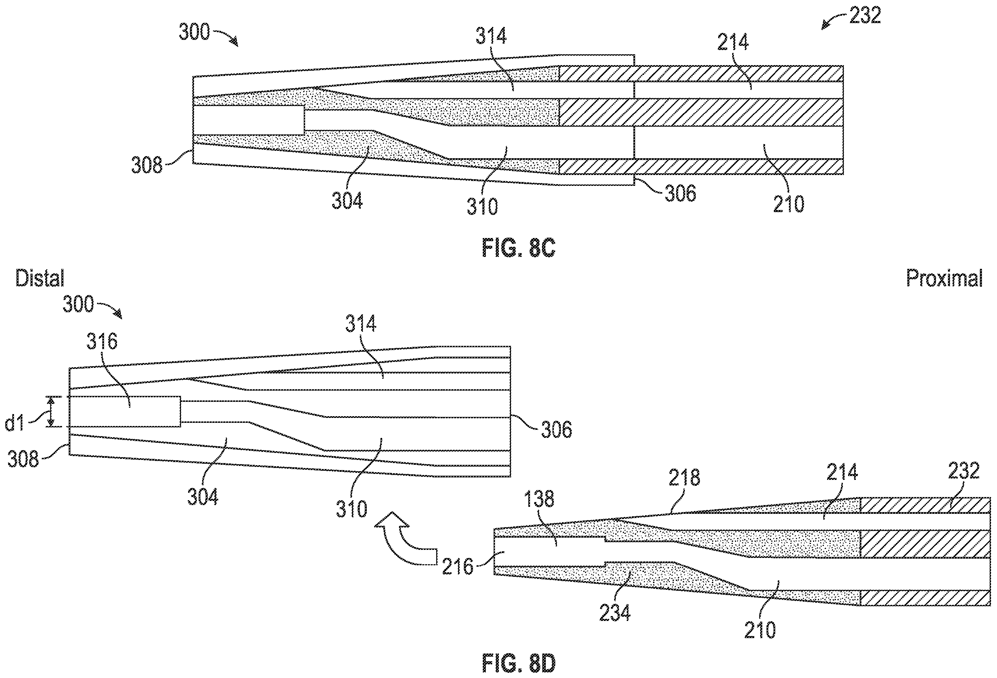

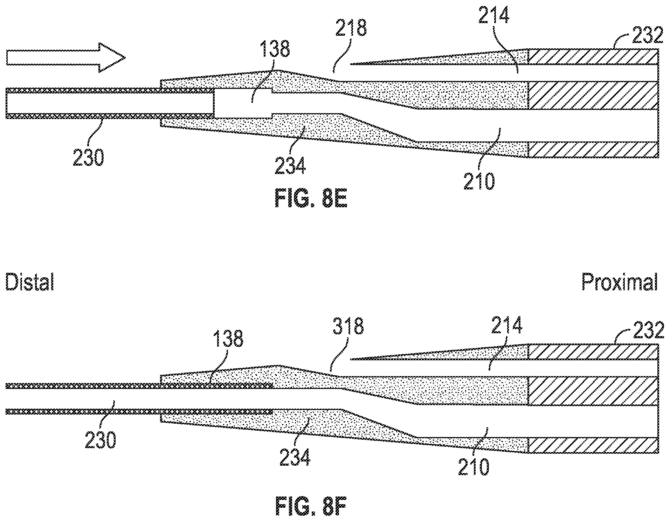

The dilator section 134 of the catheter tube 124 couples the access section 130 and the catheter body section 132 of the catheter tube 124 together. The dilator section 134 includes a receptacle 138 (*see* FIG. 8D) in a distal portion including the proximal portion of the access section 130 of the catheter tube 124 disposed therein and fixedly coupled (e.g., solvent bonded, welded, adhered, etc.) thereto. The proximal end of the dilator section 134 is integral (e.g., RF welded) with the distal end of the catheter body section 132 of the catheter tube 124, which, in an example effectively terminates the lumens (e.g. the medial and proximal lumens 112, 114), of the catheter body section 132 of the catheter tube 124 other than the primary lumen 110 from passing through the dilator section 134.

In an example, the dilator section 134 also includes a taper over its length from a distal end to the proximal end configured for immediate dilation of tissue about the percutaneous puncture, up to an outer diameter of the catheter body section 132 of the catheter tube 124. An abluminal surface of the dilator section 134 smoothly transitions from an abluminal surface of the proximal portion of the access section 130 without an edge that catches on skin when the RICC 102 is inserted into an insertion site of a patient. In addition to the edge being minimal to negligible, the edge can include solvent-interdiffused polymeric material of the first polymeric material and the polymeric material of the dilator section 134, which smoothens the transition from the access section 130 of the catheter tube 124 to the dilator section 134.

In an example, the dilator section 134 of the catheter tube 124 is formed of the second polymeric material or a third polymeric material having a third durometer closer to the second durometer than the first durometer. Again, the second polymeric material can be polyvinyl chloride, polyethylene, polyurethane, or silicone, but the second polymeric material is not limited to the foregoing polymers.

Again, the access section 130 of the catheter tube 124 is formed of a first polymeric material having a first durometer, the catheter body section 132 of the catheter tube 124 is formed of a second polymeric material having a second durometer less than the first durometer, and the dilator section 134 of the catheter tube 124 is formed of the second polymeric material or a third polymeric material having a third durometer closer to the second durometer than the first durometer. Being that each durometer of the second durometer and the third durometer is less than the first durometer, the soft portion of the catheter tube 124 including the second portion of the catheter tube 124 and the dilator section 134 is softer than the hard portion of the catheter tube 124 including the access section of the catheter tube 124. In other words, the first durometer is greater than each durometer of the second durometer and the third durometer. Being that the first durometer is greater than each durometer of the second durometer and the third durometer, the hard portion of the catheter tube 124 including the access section of the catheter tube 124 is harder than the soft portion of the catheter tube 124 including the second portion of the catheter tube 124 and the dilator section 134.

It should be understood the first durometer of the first polymeric material, the second durometer of the second polymeric material, and the third durometer of the third polymeric material can be on different scales (e.g., Type A or Type D), so the second durometer or the third durometer might not be numerically less than the first durometer. In other words, the first durometer material might not be numerically greater than the second durometer or the third durometer in view of the different scales. That said, the hardness of the second or third polymeric material can still be less than the hardness of the first polymeric material or the hardness of the first polymeric material can still be greater than the hardness of the second or third polymeric material because the different scales-each of which ranges from 0 to 100-are designed for characterizing different materials in groups of the materials having a like hardness.

Notwithstanding the foregoing, the access section 130 of the catheter tube 124, the catheter body section 132 of the catheter tube 124, and the dilator section 134 can be formed of a same polymeric material or different polymeric materials having substantially equal durometers provided the column strength of the catheter tube 124 is sufficient to prevent buckling of the catheter tube 124 when inserted into an insertion site established by a percutaneous puncture and advanced through a vasculature of a patient.

In an example, each extension leg of the one-or-more extension legs 128 typically includes a Luer connector coupled to the extension leg, through which Luer connector, the extension leg, and the extension-leg lumen thereof can be connected to another medical device. In an embodiment, the introducer needle 104 includes a shaft 142, a needle hub 144 about a proximal portion of the shaft 142, and a beveled tip 146 in a distal portion of the shaft 142.

When the system 100 is in the ready-to-deploy state, the introducer needle 104 or the shaft 142 thereof is disposed in the introducing lumen through the introducing aperture 122 (if present), such that the beveled tip 146 of the introducer needle 104 extends past the distal end of the access section 130 of the catheter tube 124 for establishing a percutaneous puncture. The access guidewire 106 includes a length sufficient for advancing the access guidewire 106 a sufficient distance into a blood-vessel lumen upon establishing access thereto for advancing the distal portion of the RICC 102 into the blood-vessel lumen for maintaining access.

The access guidewire 106 is disposed in a needle lumen of the introducer needle 104 when the system 100 is in at least the ready-to-deploy state. Indeed, a distal end of the access guidewire 106 is proximal of the beveled tip 146 of the introducer needle 104 but distal of the dilator section 134, which allows for immediate advancement of the distal end of the access guidewire 106 beyond the beveled tip 146 of the introducer needle 104 and into a blood-vessel lumen upon establishing access thereto.

The access guidewire 106 includes a stop 148 (e.g., a hub, a ball, a slug, etc.) about a proximal portion of the access guidewire 106 forming a stop end (e.g., a hub end, a ball end, a slug end, etc.) of the access guidewire 106. The stop end of the access guidewire 106 is larger than any aperture of the RICC 102 or the catheter tube 124 thereof, thereby providing a distal limit for advancing the access guidewire 106 into the RICC 102. The placement guidewire 108 includes an atraumatic tip (e.g., a coiled or partially coiled tip) and a length sufficient for advancing the placement guidewire 108 to the lower ⅓ of the superior vena cava ("SVC") of the heart.

When the system 100 is in at least the ready-to-deploy state, the placement guidewire 108 is disposed in the primary lumen 110 of the RICC 102 such that a distal end of the placement guidewire 108 is proximal of the introducing aperture 122 but distal of the catheter hub 126, which allows for immediate advancement of the distal end of the placement guidewire 108 into the blood-vessel lumen upon removing the introducer needle 104 or the shaft 142 thereof from the introducing lumen. Indeed, the placement guidewire 108 cannot be distally advanced into the introducing lumen due the presence of the introducer needle 104 or the shaft 142 thereof in at least the ready-to-deploy state of the system 100.

The placement guidewire 108 includes a stop 150 (e.g., a hub, a ball, a slug, etc.) about a proximal portion of the placement guidewire 108 forming a stop end (e.g., a hub end, a ball end, a slug end, etc.) of the placement guidewire 108. The stop end of the placement guidewire 108 is larger than a proximal end opening in the Luer connector of the extension leg in which the placement guidewire 108 is disposed, thereby providing a distal limit for advancing the placement guidewire 108 into the RICC 102.

FIGS. 4A-4B show an embodiment of a RICC 202 configured to use with a RICC system 100 as described herein. The RICC 202 generally includes a monoluminal access section 230 disposed distally and defining a first diameter (*d1*), and a multiluminal catheter body section 232 disposed proximally and defining a second diameter (*d2*). The first diameter (*d1*) is less than the second diameter (*d2*). The RICC 202 further includes a dilator section 234 disposed between the access section 230 and the catheter body section 232 and defines a tapered abluminal surface extending between the first diameter (*d1*) and the second diameter *(d2),* as described herein. In the present invention, one or more of the medial or secondary lumen aperture 218, and the proximal or tertiary lumen aperture 220 are formed within the tapered dilator section 134. In an embodiment, the secondary lumen aperture 218 and the tertiary lumen aperture 220 are disposed at an equal longitudinal length (*L4*) from the primary lumen aperture 216. In an embodiment, the length (*L4*), is between 0.1cm and 7cm. However, it will be appreciated that greater or lesser distances are also contemplated. In an embodiment, an introducing aperture 122 is disposed proximally of the dilator section 234, i.e. proximally of the secondary lumen aperture 218 and the tertiary lumen aperture 220 and communicates with the primary lumen 210.

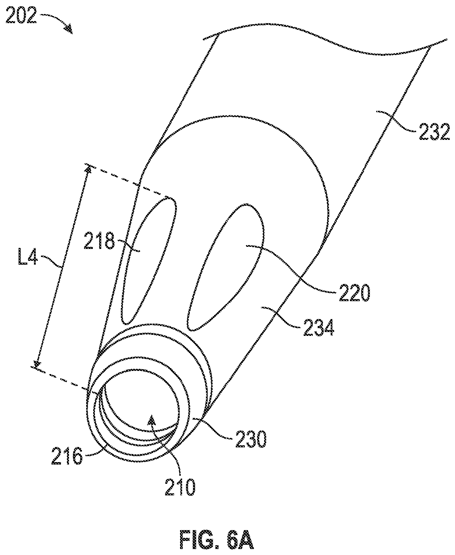

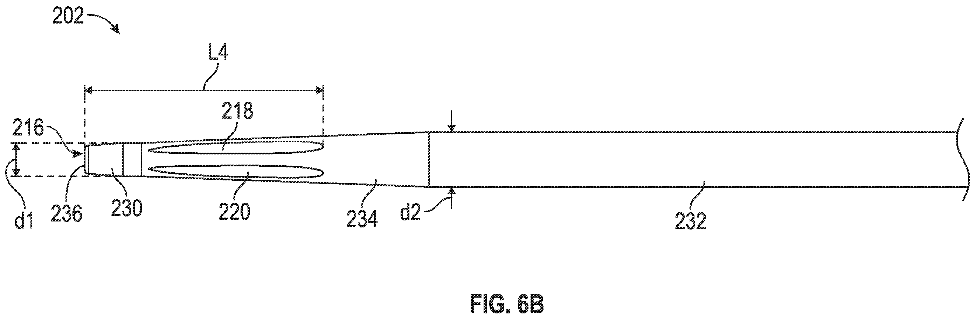

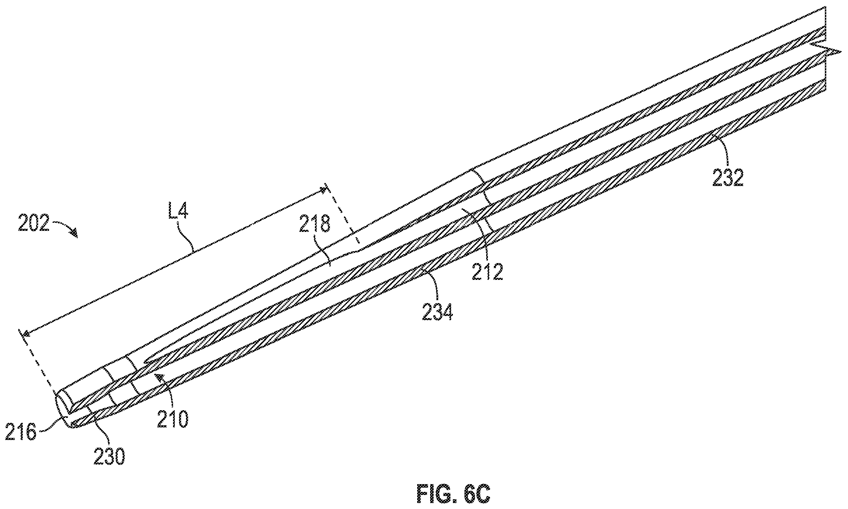

Advantageously, as shown in FIGS. 6A-6C, with the secondary lumen aperture 218 and the tertiary lumen aperture 220 disposed in the tapered dilator section 234, and the introducing aperture 122 disposed proximally of the dilator section 234, the longitudinal length of the access section 230 can be shortened to reduce the distance (*L4*) between the primary lumen aperture 216 and one or both of the secondary lumen aperture 218 and the tertiary lumen aperture 230. This can reduce the distance between an introduction site of a first fluid introduced at the primary lumen aperture 216, and a second or third fluid introduced at the secondary lumen aperture 218 or the tertiary lumen aperture 220, respectively. The positioning of the distal tip of the RICC catheter 202 within the vasculature can be critical. Where a fluid is introduced at an aperture, e.g. the secondary lumen aperture 218 or the tertiary lumen aperture 220, which is placed proximally of the target location within the vasculature, the effectiveness of the treatment is reduced. Similarly, where a distal tip 236 of the catheter 202, substantially at the primary lumen aperture 216, is advanced too far, (i.e. distally of the target location) the distal tip 236 can cause complications to the patient. For example, the distal tip 236 can be advanced into an atrium of the heart causing arrhythmia. As such, a reduced longitudinal distance between the primary lumen aperture 216 and one or both of the secondary lumen aperture 218 and the tertiary lumen aperture 220 can increase the accuracy of placement of the fluids at a target location within the vasculature.

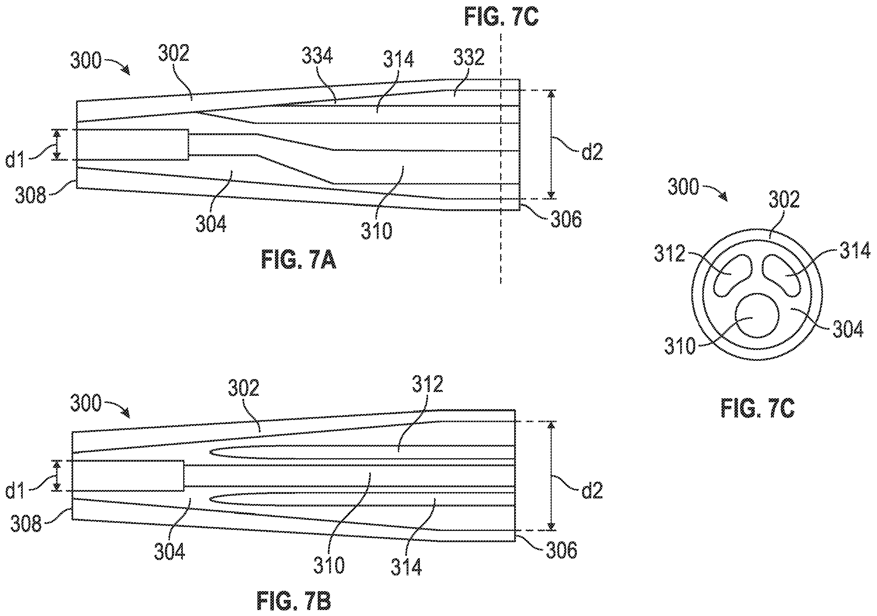

FIGS. 7A-7C show details of a die 300 used in a manufacturing process of the present invention to form the dilation section 234 of the RICC catheter 202, as described herein. The die 300 generally includes a body 302 defining a cavity 304 that extends between a proximal end 306 of the body 302 and a distal end 308 of the body 302. An interior surface of the cavity 304 defines the shape that matches an abluminal surface of the dilator section 234. The cavity 304 includes a tapered cavity 334 defining a tapered inner profile. In the invention, the tapered cavity 334 extends between the first diameter (*d1*) and the second diameter (*d2*)*.* As such, the tapered inner profile of the tapered cavity 334 matches the tapered outer profile of a dilator section 234 of the RICC 202.

In an embodiment, the cavity 304 further includes a catheter section 332 defining a cylindrical inner profile. An inner diameter of the catheter section 332 can match the outer diameter of a catheter body 232 of the RICC 202, i.e. the second diameter (*d2*). In an embodiment, the cavity 304 further includes an access section cavity (not shown) defining a cylindrical inner profile. An inner diameter of the access section cavity matches the outer diameter of the access section 230 of the RICC 202, i.e. the first diameter (*d1*)*.*

In the present invention, the die 300 further includes three mandrels extending substantially longitudinally through cavity 304 and shaped to define a profile of a portion of one or more lumens 210, 212, 214, as described in more detail herein. The die 300 includes a primary mandrel 310 extending between a proximal end 306 and a distal end 308 thereof and configured to define a portion of the primary lumen 310. The die 300 further includes one of a secondary mandrel 312 and a tertiary mandrel 314 extending from an inner surface of the cavity 304 and extending to a proximal end 306 of the body 302 and configured to define a portion of one of a secondary lumen 212 and a tertiary lumen 214, respectively. One of a secondary mandrel 312 and a tertiary mandrel 314 extends from a wall of the tapered cavity 334 of the cavity 304. In the present invention, the secondary mandrel 312 and the tertiary mandrel 314 are configured to form the secondary lumen aperture 218 and the tertiary lumen aperture 220, as described in more detail herein.

### Methods

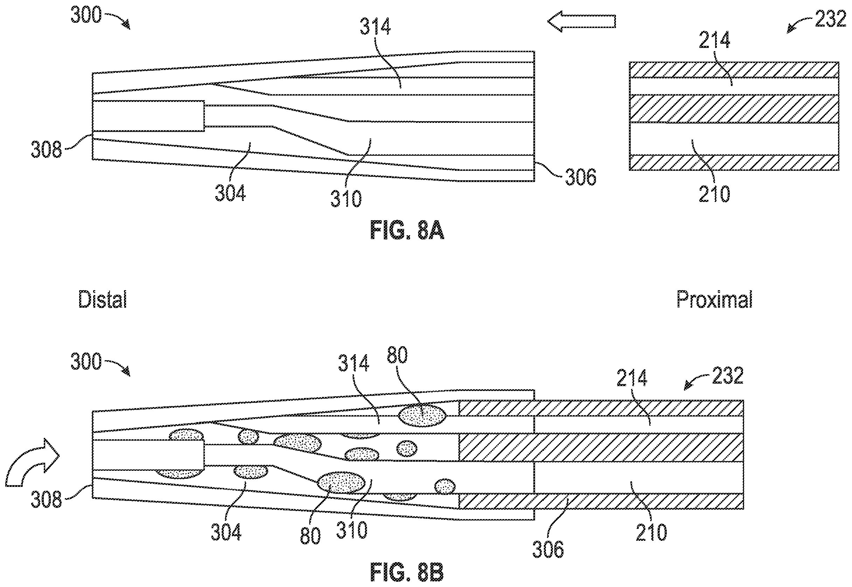

FIGS. 8A-8F show an exemplary method of forming a catheter tip for a RICC 202, termed "tipping." Advantageously, the tipping process can form a portion of distal end of the RICC 202, including one or more of the access section 230, the catheter body 232, the dilator section 234, or combinations thereof, formed of one or more of the first material, second material, or third material, or combinations thereof, as described herein. Each of the access section 230, the catheter body 232, and the dilator section 234 can display different mechanical properties and perform different functions in the placement procedure and can maintain a smooth abluminal surface therebetween. Further, embodiments described herein provide a smooth transition between inner surfaces of one or more lumen 210, 212, 214. This eliminates difficulties with guidewires catching between luminal surfaces of the access section 230, the dilator section 134, and the catheter body section 232.

As shown in FIG. 8A, initially a catheter body section 232 can be provided by obtaining or forming the catheter body section 232 of the RICC 202. The catheter body section 232 can be formed of the second polymeric material having the second durometer. The catheter body section 232 can be formed, for example, by extruding the catheter body section 232, and cutting the catheter body section 232 to an appropriate length. The catheter body section 232 can be monoluminal or multiluminal with one or more additional lumens to the primary lumen 210.

FIG. 8A also shows a die 300 including the one or more mandrels 310, 312, 314, as described herein. A distal end of the catheter body section 232 is inserted longitudinally into a proximal end of the cavity 304. More specifically, the distal end of the catheter body section 232 is received within the catheter section 332 of the cavity 304. An inner diameter of the catheter section 332 of the cavity 304 is equal to, or slightly smaller than, an outer diameter of the distal end of the catheter body section 232 to engage the catheter body section 232 in an interference fit.

In an embodiment, one or more of the mandrels 310, 312, 314, extends to a proximal end 306 of the die 300. In an embodiment, one or more of the mandrels 310, 312, 314, extends to a point that is distal of a proximal end 306 of the die 300. In an embodiment, a proximal end of one of the mandrels 310, 312, 314, engages a distal end of one of the lumens 210, 212, 214 in an interference fit and retains the catheter body section 232 within the die 300. For example a primary mandrel 310 engages a primary lumen 210 of the catheter body 232, a secondary mandrel 312 engages a secondary lumen 212 of the catheter body 232, and a tertiary mandrel 314 engages a tertiary lumen 214 of the catheter body 232. In an embodiment, a cross-sectional shape of the mandrel 310, 312, 314 is equal to, or slightly larger than, a cross-sectional shape of a respective lumen 210, 212, 214 to engage the respective lumen in an interference fit.

As shown in FIG. 8B, with the proximal end of the catheter body section 232 engaged with the die 300, a material 80, for example one of a first polymeric material, second polymeric material, or a third polymeric material, can be introduced into the cavity 304. The polymeric material can be provided as a single slug, tube, rod, or cylinder of material, or as a powder or granulated material in a flowable, or semi-flowable state. In an embodiment, the material 80 can placed within the cavity 304 before the catheter body 232 engages the catheter section 332 of the die 300. In an embodiment, the material 80 can be introduced into the cavity 304 after the catheter body 232 engages the catheter section 332 of the die 300. For example through a distal end 306 of the die 300 or through a dedicated lumen extending through a wall of the die body 302 and communicating with the cavity 304.

As shown in FIG. 8C, energy (RF energy, thermal energy, or the like) can be applied to the material 80 within the cavity 304 of the die 300 to melt the material 80 and form the catheter tip. The material 80 can conform to the shape of the cavity 304 and can conform around the mandrels 310, 312, 314. Further the material 80 can fuse with a distal end of the catheter body 232. As such, the material 80 is formed into a dilator section 234 of the RICC 202 and coupled with the distal end of the catheter body 232. Further, the join between transition section 234 and the catheter body 232 provides a smooth abluminal surface.

As shown in FIGS. 8C-8D, once the material 80 has been conformed to the shape of the cavity 304, the material 80 can be set or cured to maintain the shape. The die body 302 and the one or more mandrels 310, 312, 314 can then be removed providing the dilator section 234 coupled with the catheter body section 232 and forming a continuous abluminal surface. Further, a portion of one of the primary lumen 210, secondary lumen 212, or tertiary lumen 214 can be formed extending through the dilator section 234.

In an embodiment, the cavity 304 of the die can define the access section 230, or at least a portion of the access section 302, of the RICC 202. As such, the material 80, which can include the first material, second material, or third material, can be injected into the cavity 304, as described herein to form both the dilator section 234 and the access section 230.

In an embodiment, a diameter of the distal end 306 of the cavity 304 can be equal to an outer diameter (*d1*) of the access section 230. Further, an outer diameter of the distal end of the primary mandrel 310 can be equal to an inner diameter of the primary lumen 210. As such an access section 230 can be coupled with a distal end of the dilator section 234 by adhering, bonding, welding, or the like. In an embodiment, the primary mandrel 310 can include a collar portion 316. In an embodiment, the outer diameter of the collar 316 of the primary mandrel 310 can be equal to an outer diameter (*d1*) of the access section 230.

As such, as shown in FIGS. 8E-8F, a proximal end of the access section 230 can be inserted into a receptacle 138 formed in a distal end of the dilator section 234 and can be secured therein using adhesive, bonding, welding, or the like, as described herein. As noted, the access section 230 and the dilator section 234 can be formed of the same material and can be formed integrally within the die as a single monolithic piece and coupled to the catheter body 232. In an embodiment, the access section 230 and the dilator section 234 can be formed of the same material and can be coupled together, e.g. within the receptacle 138, using adhesive, bonding, welding, or the like, as described herein. In an embodiment, the access section 230 and the dilator section 234 can be formed of different materials and can be coupled together, e.g. within the receptacle 138, using adhesive, bonding, welding, or the like, as described herein. In an embodiment, an insertion aperture 122 can be formed in a distal portion of the catheter body 232 and communicate with the primary lumen 210.

Advantageously, methods described herein can form one or both of the secondary lumen aperture 218 or the tertiary lumen aperture 220 within the same step as forming the dilator section 234. This can remove the need for forming (e.g. machining, milling, skiving, cutting, etc.) the secondary lumen aperture 218 or the tertiary lumen aperture 220 after the formation of the dilator section 234 or after the tipping process, reducing the complexity of manufacturing and reducing associated costs. Further, embodiments, described herein show a method of forming both of the secondary lumen aperture 218 and the tertiary lumen aperture 220 at an equal longitudinal distal from a primary lumen aperture 216 and formed within the tapered profile of the dilator section 234 while maintaining a smooth abluminal and/or intra-luminal surface. Further, as shown in FIGS. 8B-8C, the die 300, including one or more of the mandrels 310, 312, 314, extend across the join between the dilator section 234 and the catheter body section 232 providing smooth abluminal and intra-lumina surfaces at the join between these two structures.

An exemplary method of using the system 100 including one of the RICC 102 or RICC 202 includes an insertion site-creating step, a RICC-inserting step, and a RICC-advancing step. The insertion site-creating step includes creating an insertion site to access a vasculature of a patient with the introducer needle 104 disposed within the primary lumen 210 of the RICC 202. The insertion site can be at a subclavian vein such as a right or left subclavian vein, an internal jugular vein such as a right or left internal jugular vein, or a femoral vein.

The RICC-inserting step includes inserting distal portion of the RICC 202 into the insertion site up to past the dilator section 234, disposed between an access section 230 and a catheter body section 232. Optionally, the RICC-inserting step is prefaced by an access guidewire-inserting step and the following needle-withdrawing step. The access guidewire-inserting step includes inserting the access guidewire 106 through the distal end of the introducer needle 104 and into one of the foregoing veins.

The method further includes a needle-withdrawing step. The needle-withdrawing step includes withdrawing the introducer needle 104 from the primary lumen 210 of the RICC 202 after the insertion site-creating step and inserting at least some of the distal portion of the RICC 202 into the insertion site.

The RICC-advancing step includes advancing the distal portion RICC 202 through the vasculature of the patient without use of a Seldinger technique. For example, if the insertion site is at the right subclavian vein or the right internal jugular vein, the RICC-advancing step can include advancing the distal portion of the RICC 202 through the right subclavian vein or the right internal jugular vein, a right brachiocephalic vein, and into a superior vena cava. Other insertions sites such as at the left subclavian vein or the left internal jugular vein require advancing the distal portion of the RICC 202 through corresponding vasculature. Optionally, the RICC-advancing step is prefaced by a maneuver guidewire-inserting step of inserting the placement guidewire 108 through the distal end of the RICC 202 and to a target location (e.g., superior vena cava).

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the defined claims.

## Claims

1. A method of forming a rapidly insertable central catheter (202), comprising:
providing a catheter body section (232) having a primary lumen (210), a secondary lumen (212) and a tertiary lumen (213), each extending longitudinally;
placing a distal end of the catheter body within a die (300), the die having a first mandrel (310) engaging the primary lumen (210), a second mandrel (312) engaging the secondary lumen (212), and a third mandrel (314) engaging the tertiary lumen (213);
melting a polymeric material disposed within a cavity of the die (300) to form a dilator section (234) coupled to the distal tip of the catheter body (232), the dilator section (234) defining a tapered out profile and including a secondary lumen aperture (218) disposed in a side wall and communicating with the secondary lumen (212), and a tertiary lumen aperture (220) disposed in a side wall and communicating with the tertiary lumen (213).

2. The method according to claim 1, further including forming an access section (230) integrally with the dilator section (234) and extending from a distal end thereof, the access section defining a single lumen communicating with primary lumen (210) and extending to a primary lumen aperture disposed at a distal tip thereof.

3. The method according to claim 2, wherein the access section (230) and the dilator section (234) are formed of a first polymeric material and the catheter body (232) is formed of a second polymeric material, the first polymeric material displaying a harder durometer relative to the second polymeric material.

4. The method according to claim 1, wherein the first mandrel (310) includes a collar (316) disposed at a distal end thereof, the collar (316) defining an outer diameter equal to an outer diameter of an access section (230).

5. The method according to claim 4, further including placing a proximal end of the access section (230) within a receptacle of the dilator section (234), defined by the collar (316) of the first mandrel (310) and securing the access section to the dilator section using adhesive, bonding, solvent bonding, or welding.

6. The method according to claim 5, wherein the access section (310) is formed of a first polymeric material and the catheter body (232) is formed of a second polymeric material, the first polymeric material displaying a harder durometer relative to the second polymeric material, optionally
wherein the dilator section (234) is formed of one of the first polymeric material, the second polymeric material, or a third polymeric material having a harder durometer relative to the second polymeric material and a softer durometer relative to the first polymeric material.

7. The method according to any of the preceding claims, wherein the melting step further includes applying one of radio frequency (RF) energy or thermal energy to melt the polymeric material.

8. The method according to any of the preceding claims, wherein one or both of the secondary lumen aperture (218) and the tertiary lumen aperture (220) are disposed in a longitudinal mid-point of the dilator section (234) between a proximal end and a distal end.

9. The method according to any of the preceding claims, wherein the secondary lumen aperture (218) and the tertiary lumen aperture (220) are disposed at an equal longitudinal length relative to a distal tip of the rapidly insertable central catheter.

10. The method according to any of the preceding claims, further including forming an introducing aperture (122) disposed proximally of one or both of the secondary lumen (218) aperture and the tertiary lumen aperture (220) and communicating with the primary lumen (210), the introducing aperture configured to receive an access needle therethrough, a distal tip of the access needle extending through a primal lumen aperture disposed at a distal tip of the rapidly insertable central catheter, and/or
wherein providing a catheter body further includes extruding or trimming the catheter body section (232) to a predetermined length and coupling a proximal end of the catheter body to one or more of a catheter hub (126) and one or more extension legs (128).

11. A rapidly insertable central catheter system (100), comprising:
a rapidly insertable central catheter (202) comprising:
a catheter body (232) defining a primary lumen (210), a secondary lumen (212), and a tertiary lumen (214), and including an introducing aperture (122) disposed in a side wall and communicating with the primary lumen;
a dilator section (234) extending distally from the catheter body and defining a tapered outer profile, the dilator section defining a secondary lumen aperture (218) disposed in a side wall and communicating with the secondary lumen and defining a tertiary lumen aperture (220) disposed in a side wall and communicating with the tertiary lumen; and
an access section (230) extending from the dilator section (234) and including a primary lumen aperture (216) disposed at a distal tip thereof and communicating with the primary lumen; and
a needle (104) extending through the introducing aperture (122) and through a distal portion of the primary lumen, a distal tip of the needle extending through the primary lumen aperture.

12. The rapidly insertable central catheter system according to claim 11, wherein the secondary lumen aperture (218) and the tertiary lumen aperture (220) are disposed at an equal longitudinal length from a distal tip of the access section (230).

13. The rapidly insertable central catheter system according to any of claims 11-12, wherein a longitudinal length of the access section (230) is less than a longitudinal length of the dilator section (234).

14. The rapidly insertable central catheter system according to any of claims 11-13, wherein the access section (230) is formed of a first material and the catheter body (232) is formed of a second material having a softer durometer relative to the first material, and/or
wherein the dilator section (234) is formed of one of the first material, the second material or a third material having a harder durometer relative to the second material and a softer durometer relative to the first material.

15. The rapidly insertable central catheter system according to any of claims 11-14, wherein the dilator section (234) and the access section (230) are formed integrally as a single monolithic piece and fused with the catheter body (232) using RF welding, and/or
wherein the dilator section (234) is fused with the catheter body (232) using RF welding and defines a receptacle (138) at a distal end, the receptacle having a diameter equal to an outer diameter of the access section, a proximal end of the access section coupled with the receptacle using adhesive, bonding, solvent bonding, or welding.

## Patentansprüche

1. Verfahren zur Bildung eines schnell einführbaren Zentralkatheters (202), umfassend:
Bereitstellen eines Katheterkörperabschnitts (232) mit einem primären Lumen (210), einem sekundären Lumen (212) und einem tertiären Lumen (213), die sich jeweils in Längsrichtung erstrecken;
Platzieren eines distalen Endes des Katheterkörpers innerhalb einer Matrize (300), wobei die Matrize einen ersten Dorn (310) aufweist, der in das primäre Lumen (210) eingreift, einen zweiten Dorn (312), der in das sekundäre Lumen (212) eingreift, und einen dritten Dorn (314), der in das tertiäre Lumen (213) eingreift;
Schmelzen eines polymeren Materials, das innerhalb eines Hohlraums der Matrize (300) angeordnet ist, um einen Dilatatorabschnitt (234) zu bilden, der mit der distalen Spitze des Katheterkörpers (232) gekoppelt ist, wobei der Dilatatorabschnitt (234) ein sich verjüngendes Profil definiert und eine in einer Seitenwand angeordnete und mit dem sekundären Lumen (212) in Verbindung stehende sekundäre Lumenöffnung (218) sowie eine in einer Seitenwand angeordnete und mit dem tertiären Lumen (213) in Verbindung stehende tertiäre Lumenöffnung (220) einschließt.

2. Verfahren nach Anspruch 1, weiter einschließlich des Bildens eines Zugangsabschnitts (230), der integral mit dem Dilatatorabschnitt (234) ausgebildet ist und sich von dessen distalem Ende erstreckt, wobei der Zugangsabschnitt ein einzelnes Lumen definiert, das mit dem primären Lumen (210) in Verbindung steht und sich zu einer primären Lumenöffnung erstreckt, die an dessen distaler Spitze angeordnet ist.

3. Verfahren nach Anspruch 2, wobei der Zugangsabschnitt (230) und der Dilatatorabschnitt (234) aus einem ersten polymeren Material gebildet sind und der Katheterkörper (232) aus einem zweiten polymeren Material gebildet ist, wobei das erste polymere Material im Vergleich zu dem zweiten polymeren Material eine höhere Härte aufweist.

4. Verfahren nach Anspruch 1, wobei der erste Dorn (310) einen Kragen (316) einschließt, der an seinem distalen Ende angeordnet ist, wobei der Kragen (316) einen Außendurchmesser definiert, der dem Außendurchmesser eines Zugangsabschnitts (230) entspricht.

5. Verfahren nach Anspruch 4, weiter einschließlich des Platzierens eines proximalen Endes des Zugangsabschnitts (230) innerhalb einer Aufnahme des Dilatatorabschnitts (234), die durch den Kragen (316) des ersten Dorns (310) definiert ist, und des Befestigens des Zugangsabschnitts an dem Dilatatorabschnitt unter Verwendung von Klebstoff, Kleben, Lösungsmittelkleben oder Schweißen.

6. Verfahren nach Anspruch 5, wobei der Zugangsabschnitt (310) aus einem ersten polymeren Material gebildet ist und der Katheterkörper (232) aus einem zweiten polymeren Material gebildet ist, wobei das erste polymere Material im Vergleich zu dem zweiten polymeren Material eine höhere Härte aufweist, optional
wobei der Dilatatorabschnitt (234) aus einem von dem ersten polymeren Material, dem zweiten polymeren Material oder einem dritten polymeren Material gebildet ist, das im Vergleich zu dem zweiten polymeren Material eine höhere Härte aufweist und im Vergleich zu dem ersten polymeren Material eine geringere Härte aufweist.

7. Verfahren nach einem der vorstehenden Ansprüche, wobei der Schmelzschritt weiter das Aufbringen von entweder Hochfrequenz- (HF) -Energie oder Wärmeenergie zum Schmelzen des polymeren Materials einschließt.

8. Verfahren nach einem der vorstehenden Ansprüche, wobei eine oder beide der sekundären Lumenöffnung (218) und der tertiären Lumenöffnung (220) in einem Längsmittelpunkt des Dilatatorabschnitts (234) zwischen einem proximalen Ende und einem distalen Ende angeordnet sind.

9. Verfahren nach einem der vorstehenden Ansprüche, wobei die sekundäre Lumenöffnung (218) und die tertiäre Lumenöffnung (220) in einer gleichen longitudinalen Länge relativ zu einer distalen Spitze des schnell einführbaren Zentralkatheters angeordnet sind.

10. Verfahren nach einem der vorstehenden Ansprüche, weiter einschließlich des Bildens einer Einführöffnung (122), die proximal von einer oder beiden der sekundären Lumen- (218) -Öffnung und der tertiären Lumenöffnung (220) angeordnet ist und mit dem primären Lumen (210) in Verbindung steht, wobei die Einführöffnung so konfiguriert ist, dass sie eine Zugangsnadel aufnehmen kann, wobei sich eine distale Spitze der Zugangsnadel durch eine primäre Lumenöffnung erstreckt, die an einer distalen Spitze des schnell einführbaren Zentralkatheters angeordnet ist, und/oder
wobei das Bereitstellen eines Katheterkörpers weiter das Extrudieren oder Trimmen des Katheterkörperabschnitts (232) auf eine vorbestimmte Länge und das Koppeln eines proximalen Endes des Katheterkörpers mit einem oder mehreren von einem Katheterknotenpunkt (126) und einem oder mehreren Verlängerungsbeinen (128) einschließt.

11. Schnell einführbares Zentralkathetersystem (100), umfassend:
einen schnell einführbaren Zentralkatheter (202), umfassend:
einen Katheterkörper (232), der ein primäres Lumen (210), ein sekundäres Lumen (212) und ein tertiäres Lumen (214) definiert und eine Einführöffnung (122) einschließt, die in einer Seitenwand angeordnet ist und mit dem primären Lumen in Verbindung steht;
einen Dilatatorabschnitt (234), der sich distal von dem Katheterkörper erstreckt und ein sich verjüngendes Außenprofil definiert, wobei der Dilatatorabschnitt eine in einer Seitenwand angeordnete und mit dem sekundären Lumen in Verbindung stehende sekundäre Lumenöffnung (218) definiert und eine in einer Seitenwand angeordnete und mit dem tertiären Lumen in Verbindung stehende tertiäre Lumenöffnung (220) definiert; und
einen Zugangsabschnitt (230), der sich von dem Dilatatorabschnitt (234) erstreckt und eine primäre Lumenöffnung (216) einschließt, die an einer distalen Spitze davon angeordnet ist und mit dem primären Lumen in Verbindung steht; und
eine Nadel (104), die sich durch die Einführöffnung (122) und durch einen distalen Abschnitt des primären Lumens erstreckt, wobei sich eine distale Spitze der Nadel durch die primäre Lumenöffnung erstreckt.

12. Schnell einführbares Zentralkathetersystem nach Anspruch 11, wobei die sekundäre Lumenöffnung (218) und die tertiäre Lumenöffnung (220) in einer gleichen longitudinalen Länge von einer distalen Spitze des Zugangsabschnitts (230) angeordnet sind.

13. Schnell einführbares Zentralkathetersystem nach einem der Ansprüche 11 - 12, wobei eine longitudinale Länge des Zugangsabschnitts (230) geringer ist als eine longitudinale Länge des Dilatatorabschnitts (234).

14. Schnell einführbares Zentralkathetersystem nach einem der Ansprüche 11 - 13, wobei der Zugangsabschnitt (230) aus einem ersten Material gebildet ist und der Katheterkörper (232) aus einem zweiten Material gebildet ist, das im Vergleich zu dem ersten Material eine geringere Härte aufweist, und/oder
wobei der Dilatatorabschnitt (234) aus einem von dem ersten Material, dem zweiten Material oder einem dritten Material gebildet ist, das im Vergleich zu dem zweiten Material eine höhere Härte und im Vergleich zu dem ersten Material eine geringere Härte aufweist.

15. Schnell einführbares Zentralkathetersystem nach einem der Ansprüche 11 - 14, wobei der Dilatatorabschnitt (234) und der Zugangsabschnitt (230) integral als ein einziges monolithisches Stück ausgebildet und mit dem Katheterkörper (232) unter Verwendung von HF-Schweißen verschmolzen sind, und/oder
wobei der Dilatatorabschnitt (234) unter Verwendung von HF-Schweißen mit dem Katheterkörper (232) verschmolzen ist und an einem distalen Ende eine Aufnahme (138) definiert, wobei die Aufnahme einen Durchmesser aufweist, der dem Außendurchmesser des Zugangsabschnitts entspricht, und wobei ein proximales Ende des Zugangsabschnitts unter Verwendung von Klebstoff, Kleben, Lösungsmittelkleben oder Schweißen mit der Aufnahme gekoppelt ist.

## Revendications

1. Procédé de formation d'un cathéter (202) central à insertion rapide, comprenant :
la fourniture d'une section (232) de corps de cathéter présentant une lumière (210) primaire, une lumière (212) secondaire et une lumière (213) tertiaire, chacune s'étendant longitudinalement ;
le placement d'une extrémité distale du corps de cathéter à l'intérieur d'une matrice (300), la matrice présentant un premier mandrin (310) venant en prise avec la lumière (210) primaire, un deuxième mandrin (312) venant en prise avec la lumière (212) secondaire, et un troisième mandrin (314) venant en prise avec la lumière (213) tertiaire ;
la fusion d'un matériau polymère disposé à l'intérieur d'une cavité de la matrice (300) pour former une section (234) de dilatateur couplée à la pointe distale du corps (232) de cathéter, la section (234) de dilatateur définissant un profil effilé et incluant une ouverture (218) de lumière secondaire disposée dans une paroi latérale et communiquant avec la lumière (212) secondaire, et une ouverture (220) de lumière tertiaire disposée dans une paroi latérale et communiquant avec la lumière (213) tertiaire.

2. Procédé selon la revendication 1, incluant en outre la formation d'une section (230) d'accès solidaire de la section (234) de dilatateur et s'étendant à partir d'une extrémité distale de celle-ci, la section d'accès définissant une lumière unique communiquant avec la lumière (210) primaire et s'étendant vers une ouverture de lumière primaire disposée à une pointe distale de celle-ci.

3. Procédé selon la revendication 2, dans lequel la section (230) d'accès et la section (234) de dilatateur sont formées d'un premier matériau polymère et le corps (232) de cathéter est formé d'un deuxième matériau polymère, le premier matériau polymère présentant un duromètre plus dur par rapport au deuxième matériau polymère.

4. Procédé selon la revendication 1, dans lequel le premier mandrin (310) inclut un collier (316) disposé à une extrémité distale de celui-ci, le collier (316) définissant un diamètre extérieur égal à un diamètre extérieur d'une section (230) d'accès.

5. Procédé selon la revendication 4, incluant en outre le placement d'une extrémité proximale de la section (230) d'accès à l'intérieur d'un réceptacle de la section (234) de dilatateur, défini par le collier (316) du premier mandrin (310) et la fixation de la section d'accès à la section de dilatateur à l'aide d'un adhésif, d'un collage, d'un collage au solvant ou d'une soudure.

6. Procédé selon la revendication 5, dans lequel la section (310) d'accès est formée d'un premier matériau polymère et le corps (232) de cathéter est formé d'un deuxième matériau polymère, le premier matériau polymère présentant un duromètre plus dur par rapport au deuxième matériau polymère, facultativement
dans lequel la section (234) de dilatateur est formée d'un parmi le premier matériau polymère, le deuxième matériau polymère, ou un troisième matériau polymère présentant un duromètre plus dur par rapport au deuxième matériau polymère et un duromètre plus doux par rapport au premier matériau polymère.

7. Procédé selon l'une quelconque des revendications précédentes, dans lequel l'étape de fusion inclut en outre l'application d'une parmi l'énergie radiofréquence (RF) ou l'énergie thermique pour faire fondre le matériau polymère.

8. Procédé selon l'une quelconque des revendications précédentes, dans lequel l'une ou les deux de l'ouverture (218) de lumière secondaire et de l'ouverture (220) de lumière tertiaire sont disposées dans un point médian longitudinal de la section (234) de dilatateur entre une extrémité proximale et une extrémité distale.

9. Procédé selon l'une quelconque des revendications précédentes, dans lequel l'ouverture (218) de lumière secondaire et l'ouverture (220) de lumière tertiaire sont disposées à une longueur longitudinale égale par rapport à une pointe distale du cathéter central à insertion rapide.

10. Procédé selon l'une quelconque des revendications précédentes, incluant en outre la formation d'une ouverture (122) d'introduction disposée proximalement de l'une ou des deux de l'ouverture (218) de lumière secondaire et de l'ouverture (220) de lumière tertiaire et communiquant avec la lumière (210) primaire, l'ouverture d'introduction étant configurée pour recevoir une aiguille d'accès à travers celle-ci, une pointe distale de l'aiguille d'accès s'étendant à travers une ouverture de lumière primaire disposée au niveau d'une pointe distale du cathéter central à insertion rapide, et/ou
dans lequel la fourniture d'un corps de cathéter inclut en outre l'extrusion ou la découpe de la section (232) de corps de cathéter à une longueur prédéterminée et le couplage d'une extrémité proximale du corps de cathéter à un ou plusieurs d'un moyeu (126) de cathéter et un ou plusieurs pieds (128) d'extension.

11. Système (100) de cathéter central à insertion rapide, comprenant :
un cathéter (202) central à insertion rapide comprenant :
un corps (232) de cathéter définissant une lumière (210) primaire, une lumière (212) secondaire et une lumière (214) tertiaire, et incluant une ouverture (122) d'introduction disposée dans une paroi latérale et communiquant avec la lumière primaire ;
une section (234) de dilatateur s'étendant distalement à partir du corps de cathéter et définissant un profil externe effilé, la section de dilatateur définissant une ouverture (218) de lumière secondaire disposée dans une paroi latérale et communiquant avec la lumière secondaire et définissant une ouverture (220) de lumière tertiaire disposée dans une paroi latérale et communiquant avec la lumière tertiaire ; et
une section (230) d'accès s'étendant à partir de la section (234) de dilatateur et incluant une ouverture (216) de lumière primaire disposée à une pointe distale de celle-ci et communiquant avec la lumière primaire ; et
une aiguille (104) s'étendant à travers l'ouverture (122) d'introduction et à travers une partie distale de la lumière primaire, une pointe distale de l'aiguille s'étendant à travers l'ouverture de lumière primaire.

12. Système de cathéter central à insertion rapide selon la revendication 11, dans lequel l'ouverture (218) de lumière secondaire et l'ouverture (220) de lumière tertiaire sont disposées à une longueur longitudinale égale à partir d'une pointe distale de la section (230) d'accès.

13. Système de cathéter central à insertion rapide selon l'une quelconque des revendications 11 à 12, dans lequel une longueur longitudinale de la section (230) d'accès est inférieure à une longueur longitudinale de la section (234) de dilatateur.

14. Système de cathéter central à insertion rapide selon l'une quelconque des revendications 11 à 13, dans lequel la section (230) d'accès est formée d'un premier matériau et le corps (232) de cathéter est formé d'un deuxième matériau présentant un duromètre plus doux par rapport au premier matériau, et/ou
dans lequel la section (234) de dilatateur est formée d'un parmi le premier matériau, le deuxième matériau ou un troisième matériau présentant un duromètre plus dur par rapport au deuxième matériau et un duromètre plus doux par rapport au premier matériau.

15. Système de cathéter central à insertion rapide selon l'une quelconque des revendications 11 à 14, dans lequel la section (234) de dilatateur et la section (230) d'accès sont formées d'un seul tenant comme une seule pièce monobloc et fusionnées avec le corps (232) de cathéter à l'aide d'une soudure RF, et/ou
dans lequel la section (234) de dilatateur est fusionnée avec le corps (232) de cathéter à l'aide d'une soudure RF et définit un réceptacle (138) à une extrémité distale, le réceptacle présentant un diamètre égal à un diamètre extérieur de la section d'accès, une extrémité proximale de la section d'accès étant couplée au réceptacle à l'aide d'un adhésif, d'un collage, d'un collage au solvant ou d'une soudure.
